# EUROPÄISCHE PATENTSCHRIFT

(11) **EP 2 155 179 B1**
(45) Veröffentlichungstag und Bekanntmachung des Hinweises auf die Patenterteilung: **01.12.2010**
(21) Anmeldenummer: 08758440.5
(22) Anmeldetag: 09.05.2008
(51) Int. Cl.: A61K 31/135, A61P 19/02, A61P 25/04

(54) **AXOMADOL ZUR SCHMERZBEHANDLUNG BEI ARTHROSE**
AXOMADOL FOR TREATING PAIN FROM ARTHRITIS
AXOMADOL POUR LE TRAITEMENT DE LA DOULEUR EN CAS D'ARTHROSE

(30) Priorität: 11.05.2007 DE 102007022790
(43) Veröffentlichungstag der Anmeldung: 24.02.2010
(73) Patentinhaber: Grünenthal GmbH, 52078 Aachen (DE)
(72) Erfinder: SCHIENE, Klaus, 41363 Jüchen (DE); BLOMS-FUNKE, Petra, 52146 Würselen (DE); BOTHMER, John, NL-6418 PR Heerlen (NL); LEFEBER, Claudia, D-52072 Aachen (DE)
(74) Vertreter: Bülle, Jan
(86) Internationale Anmeldenummer: PCT/EP2008/003755
(87) Internationale Veröffentlichungsnummer: WO 2008/138558

(56) Entgegenhaltungen:
- EP-A- 0 753 506
- WO-A-2006/089708
- DE-A1-102005 009 217
- FLEISCHMANN R M ET AL: "TRAMADOL FOR THE TREATMENT OF JOINT PAIN ASSOCIATED WITH OSTEOARTHRITIS: A RANDOMIZED, DOUBLE-BLIND, PLACEBO-CONTROLLED TRIAL" CURRENT THERAPEUTIC RESEARCH, EXCERPTA MEDICA, TRENTON, NJ, US, Bd. 62, Nr. 2, 1. Februar 2001 (2001-02-01), Seiten 113-128, XP001199507 ISSN: 0011-393X

## Beschreibung

Die Erfindung betrifft die Verwendung von Axomadol zur Behandlung von Schmerz bei Arthrose.

Arthrose (*Osteoarthritis, Arthrosis deformans*) ist die am weitesten verbreitete menschliche Gelenkerkrankung. Sie ist eine dynamische, aber langsam progrediente, degenerative Erkrankung des Knorpels und anderer Gelenkgewebe, vor allem bei älteren Individuen, mit intermittierenden entzündlichen Episoden. Aufgrund der fehlenden Entzündungsparameter, eingeschränkter Beweglichkeit, kurzfristiger Gelenksteifigkeit und radiologischer Merkmale kann sie von anderen rheumatischen Erkrankungen abgegrenzt werden.

Die Arthrose oder der Gelenkverschleiß ist ein Gelenkschaden, die mit einem Abbau des Gelenkknorpels beginnt. In schweren Fällen kommt es schließlich zu Umbauprozessen im benachbarten Knochen, die Gelenkoberfläche wird zerstört. Die Folgen der Erkrankung sind daher Schmerzen und Steifigkeit des Gelenks mit Bewegungseinschränkungen. Die Gelenke können sich verformen und schließlich ganz verknöchern. Eine Arthrose ist ein zumeist langsam fortschreitendes Geschehen. Die Knorpelschicht wird in Folge zuerst dicker und die Chondrozyten werden metabolisch aktiver. Veränderungen der subchondralen Trabekel führen zu einer verminderten Druckentlastung durch den spongiösen Knochen. Das Reparationsgewebe wird stärker belastet und mit fortschreitender Krankheitsdauer ändert sich das Gleichgewicht hinsichtlich einer Destruktion. Röntgenologisch wird eine Gelenkspaltverengung sichtbar und an den Rändern werden Osteophyten gebildet. Bezüglich weiterer Einzelheiten kann beispielsweise vollumfänglich verwiesen werden auf D. Höffler et al., AVP Therapieempfehlungen der Arzneimittelkommission der Deutschen Ärzteschaft, Arzneiverordnung in der Praxis, "Degenerative Gelenkerkrankungen", 2. Auflage 2001; und H. Bröll et al., CliniCum, Sonderausgabe September 2001, Konsensus-Statement, "Arthrose, Diagnostik & Therapie".

Prinzipiell können alle Gelenke von arthrotischen Veränderungen betroffen sein. Am meisten betroffen sind jedoch die Kniegelenke (Gonarthrose) und Hüftgelenke (Koxarthrose), auf denen viel Gewicht lastet. Häufig kommt die Erkrankung auch in den kleinen Wirbelsäulengelenken (Spondylarthrose) sowie in den Fingergelenken vor. Gemäß der ICD-10 sind Hüft- und Knie-Arthrose als primäre Knorpelerkrankungen definiert, die mit schmerzhaften Bewegungseinschränkungen (Anlaufschmerz, Belastungsschmerz) bzw. Gehbehinderungen einhergehen. Eine Entzündung, wie eine Synovitis, kann, muss aber nicht etabliert sein.

Leit- und Frühsymptom von Arthrose sind Schmerzen (Frühtrias: Anlaufschmerz, Ermüdungsschmerz, Belastungsschmerz; Spättrias: Dauerschmerz, Nachtschmerz, Muskelschmerz). Sie werden von Bewegungseinschränkungen, Wetterfühligkeit, Krepitation begleitet. Schmerzursachen bei der Arthrose resultieren vorwiegend aus Reizzuständen in periartikulären Sehnen- und Bandansätzen, sekundären Entzündungen, Gelenkkapseldehnung, Reizergüssen, Druckerhöhung im subchondralen Knochen und Mikrofrakturen.

In frühen Stadien treten Schmerzen nur bei Belastung auf und lassen bei fortgeführter Bewegung, z.B. bei längerem Gehen, nach wenigen Minuten wieder nach. Tritt eine Entzündung hinzu, zeigen sich die typischen Beschwerden der aktivierten Arthrose: das Gelenk schmerzt, fühlt sich warm an und ist geschwollen. Die Beweglichkeit ist eingeschränkt. Oft klingt die Entzündung auch ohne Behandlung ab. Dies erklärt den meist schubweisen Verlauf der Arthrose: Phasen stärkerer Schmerzen und Bewegungseinschränkung wechseln sich mit Phasen geringer Schmerzhaftigkeit und guter Beweglichkeit ab. Je weiter die Abnutzungserscheinungen fortschreiten, desto rascher folgt eine Schmerzphase auf die andere. Schließlich bleibt der Schmerz andauernd bestehen.

In der Behandlung stehen mehrere nicht-medikamentöse und medikamentöse Alternativen zur Verfügung, die einzeln oder in Kombination zur Anwendung kommen:
- allgemeine Maßnahmen, z.B. Schwimmen, Radfahren, gezielte Gymnastik, Benutzung von Gehhilfen, Diät, etc.;
- physikalische Therapie, z.B. Wärmepackungen, Elektrotherapie und Bewegungstherapie, etc.;
- Pharmakotherapie;
- Orthopädie-Technik, z.B. Bandagen, Orthesen, etc.; und
- operative Therapie, z.B. Transplantation von autologen Knorpelzellen, künstlicher Gelenkersatz, etc.

Zur Beurteilung des Erfolges einer bestimmten Therapie empfiehlt die *European League Against Rheumatism* (EULAR) den *Lequesne Index,* d.h. die Gesamtbewertung durch den Arzt und die Schmerzeinschätzung des Patienten. Von der FDA werden neben der Bewertung von Schwellung, Rötung und Druckfestigkeit des Gelenks die Bewertung des Schmerzes und der Funktion mittels des *Western-Ontario-McMaster-Universities-Osteoarthritis-Index* (WOMAC)- und des *Lequesne-Indices* empfohlen. Die *Osteoarthritis Research Society* empfiehlt für Arzneimittel, die zur symptomatischen Behandlung der Arthrose eingesetzt werden, die Skalen des WOMAC-Schmerzscores als Hauptzielkriterium, als Nebenzielkriterien den Bewegungseinschränkungsscore des WOMAC oder den *Lequesne Index,* außerdem die Gesamtbeurteilung durch den Arzt und die Patienten.

Das pharmakotherapeutische Spektrum der zur Therapie der Arthrose angebotenen Wirkstoffgruppen umfasst
- nicht-opioide Analgetika, z.B. Paracetamol;
- nichtsteroidale Antirheumatika/Antiphlogistika (NSAR), z.B. Acemetacin, Acetylsalicylsäure, Aceclofenac, Diclofenac, Ibuprofen, Ketoprofen, Mefenaminsäure, Tiaprofensäure, Indometacin, Lonazolac, Naproxen, Proglumetacin, Meloxicam, Piroxicam, Rofecoxib, Celecoxib;
- Opioidanalgetika, z.B. Dihydrocodein, Tramadol (Fleischmann et al, CURRENT THERAPEUTIC RESEARCH 2001, 62(2) S 113-128), Tilidin-Naloxon, Morphin, Buprenorphin, Oxycodon, Fentanyl und Hydromorphon;
- perkutan applizierbare Antiphlogistika und Hyperämika;
- Glucocorticosteroid-Kristallsuspensionen für intraartikuläre Injektionen; und
- weitere Wirkstoffe zur oralen oder intraartikulären Injektionen, z.B. Glucosamin, Ademetionion, Oxaceprol, Hyaluronsäure, etc.

Opioidanalgetika gehören nicht zum Routine-Repertoire der medikamentösen Behandlung von Arthrose, sind aber in bestimmten Situationen unumgänglich. Herkömmliche Opioidanalgetika zeigen allerdings zum Teil erhebliche Nebenwirkungen, insbesondere Obstipation, Übelkeit, Erbrechen, Kopfschmerzen, Sedierung, Müdigkeit, Atemdepression, Allergien und mitunter Blutdruckabfall. Diese Nebenwirkungen erschweren eine Langzeittherapie chronischer Schmerzzustände bei Arthrose. Daher ergibt sich die Indikation für eine Behandlung mit herkömmlichen Opioidanalgetika meist erst nach Ausschöpfen aller anderen therapeutischen Möglichkeiten, beispielsweise bei Patienten, die nicht operationsfähig sind, aber an massiven, auf andere analgetisch wirkende Substanzen nicht ansprechende Ruheschmerzen leiden.

Es besteht ein Bedarf an alternativen pharmakotherapeutischen Behandlungsmethoden der Arthrose, welche sich durch eine wirksame Schmerzbekämpfung und ein verbessertes Nebenwirkungsprofil auszeichnen.

Aufgabe der Erfindung war es daher Verbindungen aufzufinden, welche bei der Schmerzbekämpfung bei Arthrose wirksam sind und Vorteile gegenüber herkömmlichen Analgetika zeigen.

Diese Aufgabe wird durch den Gegenstand der Patentansprüche gelöst.

Die Erfindung betrifft die Verwendung von Axomadol zur Herstellung eines Medikaments zur Behandlung von Schmerz bei Arthrose.

Es wurde überraschend gefunden, dass Axomadol eine hervorragende Wirksamkeit bei der Behandlung von Schmerz bei Arthrose und ein verringertes Nebenwirkungsspektrum auf sich vereint. Darüber hinaus wurde gefunden, dass Axomadol im chronischen Entzündungsschmerzmodell eine bessere analgetische Wirksamkeit im Vergleich zu herkömmlichen Analgetika, wie z.B. Morphin, Oxycodon und Tramadol, zeigt.

Axomadol, d.h. (1*RS*,3*RS*,6*RS*)-6-Dimethylaminomethyl-1-(3-methoxyphenyl)-cyclohexan-1,3-diol, ist ein synthetisches, zentral wirkendes Analgetikum, welches bei der Behandlung von mäßigem bis schwerem, akutem oder chronischem Schmerz wirksam ist. Axomadol kann in Form ihrer freien Base oder als Salz oder Solvat eingesetzt werden.

EP-A 0 753 506 beschreibt die Synthese von Axomadol und Untersuchungen zu dessen analgetischer Wirksamkeit im *Tail-Flick*-Test an der Maus. Aus WO 01/57232 ist die stereoselektive Synthese des Axomadols mittels enzymatischer Razematspaltung der entsprechenden Estervorstufe bekannt. WO 02/43714 offenbart Axomadol zur Behandlung von vermehrtem Harndrang bzw. Harninkontinenz. WO 03/024444 beschreibt, dass auch eine Wirkstoff-Kombination, welche Axomadol und einen Muscarin-Antagonisten enthält, zur Behandlung der Haminkontinenz eingesetzt werden kann. WO 02/067916 beschreibt das Lösungsverhalten des AxomadolHydrochlorid-Salzes und die Löslichkeit des Axomadol-Saccharinats in Wasser. WO 2005/009329 offenbart pharmazeutische Formulierungen von Axomadol mit verzögerter Wirkstofffreisetzung.

Zum Zwecke der Beschreibung bedeutet "Axomadol" (1 RS,3RS,6RS)-6-Dimethylaminomethyl-1-(3-methoxyphenyl)-cyclohexan-1,3-diol, dessen pharmazeutisch verträglichen Salze und Solvate.

Geeignete pharmazeutisch verträgliche Salze umfassen Salze anorganischer und/oder organischer Säuren, wie z.B. Essigsäure, 2,2-Dichloressigsäure, acylierte Aminosäuren, vorzugsweise acetylierte Aminosäuren, wie z.B. N-Acetylalanin, N-Acetylcystein, N-Acetylglycin, N-Acetylisoleucin, N-Acetylleucin, N-Acetylmethionin, N-Acetylphenylalanin, N-Acetylprolin, N-Acetylserin, N-Acetylthreonin, N-Acetyltyrosin, N-Acetylvalin, Adipinsäure, Alginsäure, Ascorbinsäure, L-Asparaginsäure, Benzensulfonsäure, Benzoesäure, 4-Acetamidobenzoesäure, (+)-Camphersäure, (-)-Camphersäure, (+)-Camphersulfonsäure, (-)-Camphersulfonsäure, (+)-Campher-10-sulfonsäure, (-)-Campher-10-sulfonsäure, (±)-Campher-10-sulfonsäure, Caprinsäure, Capronsäure, Caprylsäure, Kationenaustauscher-Harze, Zimtsäure, Zitronensäure, Cyclohexylsulfaminsäure, Schwefelsäuremonododecylester, Ethan-1,2-disulfonsäure, Ethansulfonsäure, 2-Hydroxyethansulfonsäure, Ameisensäure, Fumarsäure, Schleimsäure (Mucinsäure, Galactarsäure), Gentisinsäure, Glucosemonocarbonsäure (Glucoheptonsäure), D-Gluconsäure, D-Glucuronsäure, L-Glutaminsäure, α-Oxoglutarsäure (α-Ketoglutarsäure), Hydroxyessigsäure (Glycolsäure), Hippursäure (N-Benzoylglycin), Bromwasserstoff, Chlorwasserstoff, (+)-L-Milchsäure, (±)-DL-Milchsäure, Lactobionsäure (4-O-β-D-Galactopyranosyl-D-gluconsäure), Maleinsäure, (-)-L-Äpfelsäure, Malonsäure, (±)-DL-Mandelsäure, Methansulfonsäure, Naphthalin-2-sulfonsäure, Naphthalin-2,5-disulfonsäure, 1-Hydroxy-2-naphthalincarbonsäure, Nikotinsäure, Salpetersäure, Ölsäure (Oleinsäure), Molkensäure (Orotsäure, Uracil-6-carbonsäure), Oxalsäure, Palmitinsäure, Pamoasäure (Embonsäure), Phosphorsäure, L-Pyroglutaminsäure, Salicylsäure, Acetylsalicylsäure, 4-Aminosalicylsäure, Sebacinsäure, Stearinsäure, Bernsteinsäure, Schwefelsäure, Gerbsäure (Tanninsäure), (+)-L-Weinsäure, (±)-DL-Weinsäure, Thiocyansäure, p-Toluensulfonsäure und Undecylensäure. Bevorzugte Salze sind das Hydrochlorid, das Saccharinat, das Dihydrogenphosphat, das Hydrogenphosphat und das Phosphat.

Axomadol kann auch als Mischung der Salze der vorstehend genannten organischen und anorganischen Säuren in jedem beliebigen Verhältnis vorliegen.

In einer bevorzugten Ausführungsform handelt es sich bei dem Medikament um eine feste Arzneiform. Vorzugsweise ist das Medikament zur oralen Verabreichung konfektioniert. Es sind jedoch auch andere Verabreichungsformen möglich, beispielsweise zur buccalen, sublingualen, transmucosalen, rektalen, intralumbalen, intraperitonealen, transdermalen, intravenösen, intramuskulären, intraglutealen, intrakutanen und subkutanen Applikation.

Je nach Konfektionierung enthält das Medikament vorzugsweise geeignete Zusatz- und/oder Hilfsstoffe. Geeignete Zusatz- und/oder Hilfsstoffe im Sinne der Erfindung sind alle dem Fachmann aus dem Stand der Technik bekannten Stoffe zur Erreichung galenischer Formulierungen. Die Auswahl dieser Hilfsstoffe sowie die einzusetzenden Mengen hängen davon ab, wie das Arzneimittel appliziert werden soll, d.h. oral, intravenös, intraperitoneal, intradermal, intramuskulär, intranasal, buccal oder lokal.

Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien zur rektalen Anwendung. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen.

Beispiele für Hilfs- und Zusatzstoffe für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc.

Für Suppositorien können u.a. Wachse bzw. Fettsäureester und für parenterale Applikationsmittel Trägerstoffe, Konservierungsmittel, Suspensionshilfsmittel etc. verwendet werden.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnussöl, Erdnussöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung dieser Arzneimittel und pharmazeutischen Zusammensetzungen erfolgt mit Hilfe von im Stand der Technik der pharmazeutischen Technologie wohlbekannten Mitteln, Vorrichtungen, Methoden und Verfahren, wie sie beispielsweise in *"*Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die den Wirkstoff in homogener Verteilung enthält. Unter einer homogenen Verteilung wird hier verstanden, dass der Wirkstoff gleichmäßig über die gesamte Zusammensetzung verteilt ist, so dass diese ohne weiteres in gleich wirksame Einheitsdosis-Formen, wie Tabletten, Kapseln, Dragees unterteilt werden kann. Die feste Zusammensetzung wird anschließend in Einheitsdosis-Formen unterteilt. Die Tabletten oder Pillen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an Patienten zu verabreichenden Mengen an Axomadol variieren in Abhängigkeit vom Gewicht des Patienten, von der Applikationsart und dem Schweregrad der Erkrankung. In einer bevorzugten Ausführungsform enthält das Medikament Axomadol in einer Menge von 10 bis 2.000 mg, bevorzugter 15 bis 1.000 mg, noch bevorzugter 20 bis 500 mg, bezogen auf die freie Base.

Aus oral, rektal oder perkutan anwendbaren Zubereitungsformen kann Axomadol verzögert freigesetzt werden. Bevorzugt ist das Medikament zur einmal täglichen Verabreichung, zur zweimal täglichen Verabreichung (bid) oder zur dreimal täglichen Verabreichung konfektioniert, wobei die zweimal täglichen Verabreichung (bid) besonders bevorzugt ist.

Eine verzögerte Freisetzung von Axomadol kann beispielsweise durch Retardierung mit Hilfe einer Matrix, eines Überzugs oder osmotisch wirkender Freisetzungssysteme erreicht werden (vgl. z.B. WO 2005/009329).

In einer bevorzugten Ausführungsform
- ist das Medikament zur oralen Verabreichung konfektioniert; und/oder
- ist das Medikament eine feste und/oder verpresste und/oder filmbeschichtete Arzneiform; und/oder
- setzt das Medikament Axomadol aus einer Matrix verzögert frei; und/oder
- enthält das Medikament Axomadol in einer Menge von 0,001 bis 99,999 Gew.-%, bevorzugter 0,1 bis 99,9 Gew.-%, noch bevorzugter 1,0 bis 99,0 Gew.-%, noch bevorzugter 2,5 bis 80 Gew.-%, am bevorzugtesten 5,0 bis 50 Gew.-% und insbesondere 7,5 bis 40 Gew.-%, bezogen auf das Gesamtgewicht des Medikaments; und/oder
- enthält das Medikament einen pharmazeutisch verträglichen Träger und/oder pharmazeutisch verträgliche Hilfsstoffe; und/oder
- weist das Medikament eine Gesamtmasse im Bereich von 25 bis 2.000 mg, bevorzugter 50 bis 1.800 mg, noch bevorzugter 60 bis 1.600 mg, noch bevorzugter 70 bis 1.400 mg, am bevorzugtesten 80 bis 1.200 mg und insbesondere 100 bis 1.000 mg auf, und/oder
- ist das Medikament ausgewählt aus der Gruppe bestehend aus Tabletten, Kapseln, Pellets und Granulaten.

Das Medikament kann als einfache Tablette und als überzogene Tablette (z.B. als Filmtablette oder Dragee) vorliegen. Die Tabletten sind üblicherweise rund und bikonvex, oblong Formen sind jedoch ebenfalls möglich. Granulate, Sphäroide, Pellets oder Mikrokapseln, welche in Sachets oder Kapseln gefüllt oder zu zerfallenden Tabletten verpresst sind, sind ebenfalls möglich.

Bevorzugt sind Medikamente, die wenigstens 0,001 bis 99,999 Gew.-% Axomadol enthalten, insbesondere niedrige, wirksame Dosierungen, um Nebenwirkungen zu vermeiden. Das Medikament enthält bevorzugt 0,01 Gew.-% bis 99,99 Gew.-% Axomadol, bevorzugter 0,1 bis 90 Gew.-%, noch bevorzugter 0,5 bis 80 Gew.-%, am bevorzugtesten 1,0 bis 50 Gew.-% und insbesondere 5,0 bis 20 Gew.-%.

Besonders bevorzugt handelt es sich bei dem Medikament um eine orale Darreichungsform, welche zur zweimal täglichen Verabreichung konfektioniert ist und Axomadol in einer Menge von 10 bis 2.000 mg enthält, bezogen auf die freie Base. Axomadol zeigt eine ausgeprägte antihyperalgetische Wirksamkeit, welche im *Complete Freund Adjuvant* (CFA) Tiermodell nachgewiesen wurde.

Erfindungsgemäß wird Axomadol zur Behandlung von Schmerz bei Arthrose verwendet. Bevorzugt ist die Arthrose ausgewählt aus der Gruppe bestehend aus Gonarthrose, Koxarthrose und Spondylarthrose.

Bevorzugt handelt es sich bei der schmerzenden Arthrose um Arthrose im Sinne der ICD-10 (Internationale statistische Klassifikation der Krankheiten und verwandter Gesundheitsprobleme, WHO Ausgabe, vorzugsweise Stand 2007). Bevorzugt ist die Arthrose ausgewählt aus Polyarthrose [M15], Koxarthrose [M16], Gonarthrose [M17], Rhizarthrose [M18], Sonstige Arthrose [M19] und Arthrose der Wirbelsäule [M47]. Die in Klammern angegebenen Bezeichnungen beziehen sich auf die in der ICD-1 0 verwendete Nomenklatur.

Handelt es sich bei der Arthrose um Polyarthrose [M15], so ist diese vorzugsweise ausgewählt aus der Gruppe bestehend aus primärer, generalisierter (Osteo-)Arthrose [M15.0], Heberden-Knoten (mit Arthropathie) [M15.1], Bouchard-Knoten (mit Arthropathie) [M15.2], sekundärer, multipler Arthrose (posttraumatische Polyarthrose) [M15.3], erosiver (Osteo-)Arthrose [M15.4], sonstiger Polyarthrose [M15.8] und nicht näher bezeichneter Polyarthrose (generalisierter (Osteo-)Arthrose ohne nähere Angabe) [M15.9].

Handelt es sich bei der Arthrose um Koxarthrose [M16], so ist diese vorzugsweise ausgewählt aus der Gruppe bestehend aus beidseitiger primärer Koxarthrose [M16.0], sonstiger primärer Koxarthrose (einseitig oder ohne nähere Angabe) [M16.1], beidseitiger Koxarthrose als Folge einer Dysplasie [M16.2], sonstige dysplasische Koxarthrose (einseitig oder ohne nähere Angabe) [M16.3], beidseitiger, posttraumatischer Koxarthrose [M16.4], sonstiger posttraumatischer Koxarthrose [M16.5] (einseitig oder ohne nähere Angabe), sonstiger, beidseitiger sekundärer Koxarthrose [M16.6], sonstiger sekundärer Koxarthrose (einseitig oder ohne nähere Angabe) [M16.7] und nicht näher bezeichneter Koxarthrose [M16.9].

Handelt es sich bei der Arthrose um Gonarthrose [M17], so ist diese vorzugsweise ausgewählt aus der Gruppe bestehend aus beidseitiger primärer Gonarthrose [M17.0], sonstiger primärer Gonarthrose (einseitig oder ohne nähere Angabe) [M17.1], beidseitiger, posttraumatischer Gonarthrose [M17.2], sonstiger posttraumatischer Gonarthrose [M17.3] (einseitig oder ohne nähere Angabe), sonstiger, beidseitiger sekundärer Gonarthrose [M17.4], sonstiger sekundärer Gonarthrose (einseitig oder ohne nähere Angabe) [M17.5] und nicht näher bezeichneter Gonarthrose [M17.9].

Handelt es sich bei der Arthrose um Rhizarthrose [M18], so ist diese vorzugsweise ausgewählt aus der Gruppe bestehend aus beidseitiger primärer Rhizarthrose [M18.0], sonstiger primärer Rhizarthrose (einseitig oder ohne nähere Angabe) [M18.1], beidseitiger, posttraumatischer Rhizarthrose [M18.2], sonstiger posttraumatischer Rhizarthrose [M18.3] (einseitig oder ohne nähere Angabe), sonstiger, beidseitiger sekundärer Rhizarthrose [M18.4], sonstiger sekundärer Rhizarthrose (einseitig oder ohne nähere Angabe) [M18.5] und nicht näher bezeichneter Rhizarthrose [M18.9].

Handelt es sich bei der Arthrose um Sonstige Arthrose [M19], so ist diese vorzugsweise ausgewählt aus der Gruppe bestehend aus primärer Arthrose sonstiger Gelenke (primäre Arthrose ohne nähere Angabe) [M19.0], posttraumatischer Arthrose sonstiger Gelenke (posttraumatische Arthrose ohne nähere Angabe) [M19.1], sonstiger sekundärer Arthrose (sekundäre Arthrose ohne nähere Angabe) [M19.2], sonstiger näher bezeichneter Arthrose [M19.8] und nicht näher bezeichneter Arthrose [M19.9].

Bevorzugt ist der Schmerz mäßig bis stark. In einer bevorzugten Ausführungsform ist der Schmerz ausgewählt aus der Gruppe bestehend aus Anlaufschmerz, Belastungsschmerz, Ermüdungsschmerz, periartikulärem Druckschmerz, ausstrahlendem Schmerz (z.B. Knieschmerz bei bestehender Koxarthrose), Ruheschmerz nach längerem Verharren in gleicher Stellung, Dauerschmerz, Spontanschmerz, Bewegungsschmerz, Nachtschmerz, Muskelschmerz, Endlagenschmerz, ossärem Schmerz als Spontan- und Ruheschmerz. Bevorzugt ist der Schmerz Hyperalgesie oder Allodynie. Die Hyperalgesie ist bevorzugt thermisch oder mechanisch induziert. Auch wenn die erfindungsgemäßen Medikamente lediglich geringe Nebenwirkungen zeigen, kann es beispielsweise zur Vermeidung von bestimmten Formen der Abhängigkeit von Vorteil sein, neben Axomadol auch Morphinantagonisten, insbesondere Naloxon, Naltrexon und/oder Levallorphan, zu verwenden.

Ferner betrifft die Erfindung Axomadol zur Verwendung in einem Verfahren zur Behandlung von Schmerz bei Arthrose, bei dem Axomadol in einer pharmazeutisch verträglichen Menge an einen Patienten verabreicht wird.

Die folgenden Beispiele dienen zur näheren Erläuterung der Erfindung.

### Beispiele:

### 1. Klinische Studien

Es wurden zwei Studien mit einer Behandlungsdauer von jeweils 4 Wochen zur Bestimmung der Wirksamkeit und Sicherheit von Axomadol in Patienten mit mittleren bis schweren chronischen Schmerzen aufgrund von Osteoarthritis (Arthrose, OA) der funktionellen Klasse I-III durchgeführt. Beide Studien hatten ein randomisiertes, multizentrisches, doppelblindes, Doppeldummy, placebo- und aktivkontrolliertes Parallelgruppen Studiendesign.

Patienten, die mit Axomadol behandelt wurden, zeigten eine klinisch signifikante Abnahme der Schmerzintensität nach 4 Wochen.

Während der gesamten Studiendauer traten in beiden Studien unerwünschte Wirkungen in den mit aktiver Substanz behandelten Patientengruppen häufiger auf als in den Patientengruppen, an die das Placebo verabreicht wurde. Diese unerwünschten Wirkungen sind im Allgemeinen typisch für zentral wirksame Analgetika.

### Studie A:

In dieser Studie wurden die Patienten mit Hüft- oder Knie-OA in 5 Gruppen unterteilt. Den Patienten in 3 dieser Gruppen wurden unterschiedliche Tagesdosierungen von Axomadol verabreicht (bezogen auf die freie Base 44, 66 und 110 mg je zweimal täglich), eine Gruppe bekam Tramadol (100 mg zweimal täglich) und eine Gruppe wurde mit Placebo zweimal täglich behandelt.

In allen Patientengruppen wurde zu jedem Bewertungszeitpunkt eine Abnahme der Schmerzintensität im Vergleich zum Baseline-Schmerz festgestellt. Im *full analysis set* wurde am Tag 29 eine klinisch relevante Abnahme der Schmerzintensität in den Patientengruppen festgestellt, die mit Axomadol behandelt wurden. Für die Tramadol- und die Placebogruppe wurde diese Verbesserung nicht beobachtet.

Eine klinisch relevante Abnahme der Schmerzintensität im *per protocol set* konnte in Abhängigkeit der Dosierung gezeigt werden (höhere Wirksamkeit bei höherer Dosierung) und die Ergebnisse der Patientengruppe, die 110 mg Axomadol zweimal täglich erhielt, waren statistisch signifikant (p < 0.05) im Vergleich zur Placebogruppe.

Das Auftreten der häufigsten unerwünschten Wirkungen war in der Patientengruppe, die 110 mg Axomadolhydrochlorid zweimal täglich erhielt höher als in jeder anderen Behandlungsgruppe. Die häufigsten unerwünschten Wirkungen waren Übelkeit, Verstopfung, übermäßiges Schwitzen, Schwindel, Erbrechen, Kopfschmerz, trockener Mund und Schläfrigkeit.

### Studie B:

In dieser Studie wurden die Patienten mit Knie-OA in 4 Gruppen unterteilt. Zwei der Patientengruppen erhielten unterschiedliche Dosierungen an Axomadolhydrochlorid: 100 mg und 150 mg bezogen auf die freie Base, jeweils zweimal täglich nach einer Titrationszeit von 2 Wochen, in der die Dosierung von Axomadol wöchentlich gesteigert wurde. Eine weitere Patientengruppe erhielt Oxycodon CR (20 mg zweimal täglich) nach einer Titrationszeit, in der Oxycodon CR von 10 mg auf 20 mg zweimal täglich gesteigert wurde. Eine weitere Gruppe erhielt Placebo zweimal täglich.

Die Analyse der durchschnittlichen Schmerzintensität der letzten 24 h am Tag 29 zeigte für die Patientengruppe, die 100 mg Axomadol zweimal täglich erhielt einen statistisch signifikanten Unterschied (p = 0,0190) im Vergleich zu Placebo im *full analysis set.*

Im *per protocol set* zeigten sowohl beide Axomadolgruppen (p = 0.0068 für die100 mg Axomadolgruppe und p = 0,0079 für die 150 mg Axomadolgruppe) wie auch die Oxycodongruppe (p = 0,0154) einen statistisch signifikanten Unterschied für den primären Endpunkt im Vergleich zur Placebogruppe. Verschiedene sekundäre Endpunkte bestätigten diese Resultate für das *full analysis set* als auch für das *per protocol set.*

In der Studie B traten in den drei aktiven Gruppen mehr unerwünschte Wirkungen auf als in der Placebogruppe. Diese unerwünschten Wirkungen waren für die Axomadolgruppen in den meisten Fällen leicht bis moderat. Die Häufigkeit unerwünschter Nebenwirkungen, die für den individuellen Patienten zum Abbruch der Studie führten war jedoch in der Oxycodongruppe (31,5 % der Patienten) zweimal so hoch wie in den beiden Axomadolgruppen (16,3% in der 100 mg Gruppe und 17,7% in der 150 mg Gruppe). Die häufigsten unerwünschten Wirkungen waren Verstopfung, Übelkeit, Erbrechen und trockener Mund.

### 2. Antihyperalgetische Wirkung im chronischen Entzündungsschmerzmodell

Die Untersuchung zur Bestimmung der antihyperalgetischen Wirkung von Axomadol im chronischen Entzündungsschmerz, wurde in dem *Complete Freund Adjuvant* Tiermodell (CFA) an der Ratte durchgeführt.

Ein Modell zur chronischen Entzündung stellt die durch komplettes Freund's Adjuvans (CFA) ausgelöste Monoarthritis dar. Durch Injektion einer geringen Menge von CFA (100 µg M. tuberculosis) in die Hinterpfote kommt es zu einer lokalen zeitlich auf 2-4 Wochen begrenzten Entzündungsreaktion. Die parallel auftretende

Hyperalgesie (daher auch der Name CFA-Hyperalgesie (CFA-HA), der nachfolgend verwendet wird) auf mechanische bzw. thermische Stimuli ist auf die entzündete Pfote beschränkt.

Als Versuchstiere wurden Sprague Dawley-Ratten eines kommerziellen Züchters (Janvier, Belgien) mit einem Gewicht von 140-160 g verwendet. Die CFA-HA bei der Ratte wurde durch subplantare Injektion von CFA (100 µl der 1 mg/ml Mycobakterien (M. tuberculosis, hitzegetötet) /Öl-Suspension (IFA; Difco)) in die rechte Hinterpfote (ipsilateral) induziert. Der Injektionstag wurde als Tag 0 (d 0) definiert.

Die Hyperalgesie auf einen mechanischen taktilen Stimulus wurde mittels elektronischem von Frey Messgerät bestimmt (Somedic Electronic von Frey System; Somedic Sales AB, Hörby, Schweden). Mit dem von Frey Filament wurde die Pfote subplantar gereizt. Zur Quantifizierung der Sensitivität sowohl der ipsi- als auch der contralateralen Pfote auf den mechanischen Stimulus wurde die Pfotenwegziehschwelle in Gramm des aufgebrachten Drucks angegeben. Aus den vier Messwerten pro Pfote wurde der Median gebildet. Die Wegziehschwelle der ipsi- und contralateralen Pfote wurde an Tag 1 nach CFA Injektion vor (= Vorwert) und zu verschiedenen Zeitpunkten (15, 30 und 60 Minuten) nach Substanzgabe (Messwert) bestimmt. Die Wirksamkeit einer Substanz wurde als % Inhibition der Hyperalgesie wie folgt berechnet:
% Inhibition der HA = (1 - HA Messwert / HA Vorwert) × 100
HA Vorwert = Wegziehschwelle contralateral - Wegziehschwelle ipsilateral vor Substanzgabe
HA Messwert = Wegziehschwelle contratateral - Wegziehschwelle ipsilateral nach Substanzgabe

Insgesamt wurden pro Versuchstiergruppe 10 Ratten eingesetzt. Der Mittelwert ± SEM wurde aus den Medianen der Einzeltiere berechnet. Die Signifikanzberechnung erfolgte mit Hilfe der Zwei-Faktor-Analyse der Varianz (ANOVA) für wiederholte Messungen. Im Falle eines signifikanten Behandlungseffektes erfolgte ein paarweiser Vergleich zu verschiedenen Messzeitpunkten durch einen Fisher's-Signifikanz-Test, gefolgt von einem post-hoc Dunnett-Test. Die Ergebnisse wurden als statistisch signifikant bewertet bei p < 0,05.

Für die Bestimmung der maximalen Wirksamkeit (efficacy) wurde die Substanz bis zur maximal möglichen Dosis intravenös (i.v.) appliziert. Die höchstmögliche Dosis wurde definiert als diejenige Dosis, welche noch keine die Messung beeinflussenden Nebenwirkungen sowie keinen starken antinozizeptiven Effekt auf die unbehandelte Pfote zeigte und eine weitere Dosissteigerung diesen Effekt bewirkte.

Die maximale Wirksamkeit (efficacy) wurde anhand der maximal erreichbaren Inhibition der Hyperalgesie bestimmt, die in einem Dosisbereich erreicht werden konnte, der 1. keine Überlappung mit antinociceptiven Effekten an der kontralateralen Pfote und / oder 2. keine Nebenwirkungen in dem Maße induzierte, die die Interpretation der Messwerte beeinflusste.

### Ergebnis:

Axomadol vermindert die CFA-induzierte Hyperalgesie signifikant.

Eine maximale antihyperalgetische Wirkung von 40% wurde nach intravenöser Gabe von 10 mg/kg Axomadol-HCI erreicht. Höhere Dosierungen führten zu einer Abnahme der antihyperalgetischen Wirkung auf 9% sowie einer Überlappung mit einer zusätzlichen antinozizeptiven Wirkung.

Im Vergleich zu Axomadol-HCI wurde nach Gabe der zentralwirksamen Analgetika Morphin, Oxycodon und Tramadol (s. Tabelle) eine deutlich geringere maximale Wirkung erreicht (in jedem Fall < 30 % Inhibition der Hyperalgesie).

Die Ergebnisse der Untersuchungen mit Axomadol-HCI sowie weiterer zentral wirksamer Analgetika sind in der nachfolgenden Tabelle zusammengefasst:

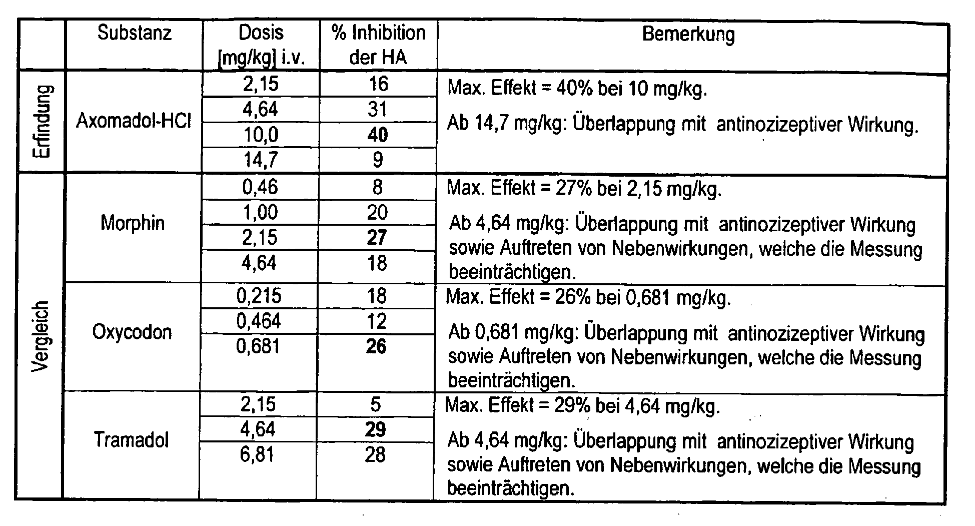

## Patentansprüche

1. Verwendung von Axomadol zur Herstellung eines Medikaments zur Behandlung von Schmerz bei Arthrose.

2. Verwendung nach dem vorstehenden Anspruch, **dadurch gekennzeichnet, dass** das Medikament eine feste Arzneiform ist.

3. Verwendung nach Anspruch 1 oder 2, **dadurch gekennzeichnet, dass** das Medikament zur oralen Verabreichung konfektioniert ist.

4. Verwendung nach einem der vorstehenden Ansprüche, **dadurch gekennzeichnet, dass** das Medikament zur zweimal täglichen Verabreichung (*bid*) konfektioniert ist.

5. Verwendung nach einem der vorstehenden Ansprüche, **dadurch gekennzeichnet, dass** das Medikament Axomadol in einer Menge von 10 bis 2.000 mg enthält.

6. Verwendung nach einem der vorstehenden Ansprüche, **dadurch gekennzeichnet, dass** Axomadol als Hydrochlorid vorliegt.

7. Verwendung nach einem der vorstehenden Ansprüche, **dadurch gekennzeichnet, dass** die Arthrose ausgewählt ist aus der Gruppe bestehend aus Gonarthrose, Koxarthrose und Spondylarthrose.

8. Verwendung nach einem der vorstehenden Ansprüche, **dadurch gekennzeichnet, dass** der Schmerz mäßig bis stark ist.

9. Verwendung nach einem der vorstehenden Ansprüche, **dadurch gekennzeichnet, dass** der Schmerz ausgewählt ist aus der Gruppe bestehend aus Anlaufschmerz, Belastungsschmerz, Ermüdungsschmerz, periartikulärem Druckschmerz, ausstrahlendem Schmerz, Ruheschmerz nach längerem Verharren in gleicher Stellung, Dauerschmerz, Spontanschmerz, Bewegungsschmerz, Nachtschmerz, Muskelschmerz, Endlagenschmerz und ossärem Schmerz als Spontan- und Ruheschmerz.

## Claims

1. Use of axomadol for the production of a medication for the treatment of pain in the case of arthrosis.

2. Use according to the preceding claim, **characterised in that** the medication is a solid drug form.

3. Use according to claim 1 or 2, **characterised in that** the medication is configured for oral administration.

4. Use according to one of the preceding claims, **characterised in that** the medication is manufactured for administration twice daily (bid).

5. Use according to one of the preceding claims, **characterised in that** the medication contains axomadol in a quantity of 10 to 2000 mg.

6. Use according to one of the preceding claims, **characterised in that** axomadol is present as hydrochloride.

7. Use according to one of the preceding claims, **characterised in that** the arthrosis is selected from the group comprising gonarthrosis, coxarthrosis and spondylarthrosis.

8. Use according to one of the preceding claims, **characterised in that** the pain is moderate to severe.

9. Use according to one of the preceding claims, **characterised in that** the pain is selected from the group comprising impact pain, weight-bearing pain, fatigue pain, periarticular pain caused by pressure, radiating pain, rest pain after remaining in the same position for a long period, continuous pain, spontaneous pain, pain on movement, night pain, muscle pain, pain in extremities and bone pain as spontaneous and rest pain.

## Revendications

1. Utilisation d'axomadol pour la préparation d'un médicament destiné au traitement de douleur en cas d'arthrose.

2. Utilisation selon la revendication précédente, **caractérisée en ce que** le médicament est une forme médicinale solide.

3. Utilisation selon la revendication 1 ou 2, **caractérisée en ce que** le médicament est confectionné pour une administration par voie orale.

4. Utilisation selon l'une quelconque des revendications précédentes, **caractérisée en ce que** le médicament est confectionné pour une administration deux fois par jour (bid).

5. Utilisation selon l'une quelconque des revendications précédentes, **caractérisée en ce que** le médicament contient de l'axomadol en une quantité de 10 à 2000 mg.

6. Utilisation selon l'une quelconque des revendications précédentes, **caractérisée en ce que** l'axomadol se trouve sous forme de chlorhydrate.

7. Utilisation selon l'une quelconque des revendications précédentes, **caractérisée en ce que** l'arthrose est choisie dans le groupe constitué par la gonarthrose, la coxarthrose et la spondylarthrose.

8. Utilisation selon l'une quelconque des revendications précédentes, **caractérisée en ce que** la douleur est modérée à forte.

9. Utilisation selon l'une quelconque des revendications précédentes, **caractérisée en ce que** la douleur est choisie dans le groupe constitué par la douleur au démarrage, la douleur de sollicitation, la douleur de fatigue, la douleur de compression péri-articulaire, la douleur rayonnante, la douleur au repos après être resté longtemps dans la même position, la douleur durable, la douleur spontanée, la douleur au mouvement, la douleur nocturne, la douleur musculaire, la douleur en position extrême et la douleur osseuse comme douleur spontanée et au repos.
